# NOUVEAU FASCICULE DE BREVET EUROPEEN

(11) **EP 0 841 942 B2**
(45) Date de publication et mention de la décision concernant l'opposition: **12.12.2007**
(45) Mention de la délivrance du brevet: 29.01.2003
(21) Numéro de dépôt: 96927102.2
(22) Date de dépôt: 29.07.1996
(51) Int. Cl.: A61K 39/12, C12N 7/02

(54) **PROCEDE DE PRODUCTION INDUSTRIELLE D'UN VACCIN CONTRE L'ENCEPHALITE JAPONAISE ET VACCIN OBTENU**
VERFAHREN ZUR INDUSTRIELLEN HERSTELLUNG EINES JAPANISCHEN ENKEPHALITIS IMPFSTOFFES, UND SO ERHALTENER IMPFSTOFF
METHOD FOR INDUSTRIALLY PRODUCING A JAPANESE ENCEPHALITIS VACCINE, AND RESULTING VACCINE

(30) Priorité: 01.08.1995 FR 9509374; 22.03.1996 FR 9603638
(43) Date de publication de la demande: 20.05.1998
(73) Titulaire: Sanofi Pasteur, 69367 Lyon Cedex 07 (FR)
(72) Inventeur: FANGET, Bernard, F-69210 Saint-Germain-sur-l'Arbresle (FR); FRANCON, Alain, F-69069 Bessenay (FR); HEIMENDINGER, Pierre, F-69008 Lyon (FR)
(74) Mandataire: Bernasconi, Jean Raymond
(86) Numéro de dépôt international: PCT/FR1996/001195
(87) Numéro de publication internationale: WO 1997/004803

(56) Documents cités:
- EP-A- 0 171 765
- EP-A- 0 468 702
- WO-A-91/09935
- WO-A-92/07084
- JP-A- 6 153 227
- BIOLOGICAL ABSTRACTS, vol. 64, 1 Juillet 1977 Philadelphia, PA, US; abstract no. 2449, C.N. VENKATESHAN ET AL.: "COMPARARTIVE STUDIES ON DIFFERENT METHODS FOR CONCENTRATION OF JAPANESE ENCEPHALITIS VIRAL ANTIGENS PREPARED FROM VERO CELL CULTURE." page 245; XP002000470 & INDIAN JOURNAL OF MEDICAL RESEARCH, vol. 64, no. 11, 1976, pages 1557-1565,
- Chen et al. Chin. J . Biolog.5(2), 1992, p.171-173
- Shi et al., Virologica Sinica, 10(4), December 1995, p. 273-277
- Memorandum from the Director of the Center for Biologics Evaluation and Research, US Department of Health and Human Services, 12 July 1993.
- Kuthan et al.,Med.Dosw.Micribiol.32, 1980, p.55-61
- Krasilnikov et al., Acta Virol.29,1985,273-278
- Crooks et al. J. Chromatography 502, 1990, p.59-68
- WHO technical reports series 747, 1987
- Gall et al., Med.Dosw.Microbiol.31, 1979, p.175-180
- Welling et al., J.Chromatography 297, 1984, p.101-109
- Krasilnikov et al., J. Chromatography 446, 1988, p. 211-219

## Description

La présente invention a trait à un procédé de production d'un vaccin pour la prévention de l'encéphalite japonaise, à base de virus de l'encéphalite japonaise (JEV) et notamment d'un vaccin utilisable chez l'homme. L'invention a également trait au vaccin obtenu par ce procédé.

Le virus de l'encéphalite japonaise, dont le vecteur de transmission est un moustique, est la cause d'infections graves, dites encéphalites japonaises, dans de nombreux pays d'Extrême-Orient ainsi que dans d'autres régions du monde.

On connaît des vaccins contre l'encéphalite japonaise qui sont obtenus par des procédés consistant à injecter le virus JEV par voie intracrânienne chez le souriceau et à récupérer les tissus infectés. L'émulsion tissulaire que l'on obtient est ensuite purifiée, généralement par des méthodes de précipitation, notamment à la protamine. D'autres techniques de purification de ces préparations tissulaires ont également été proposées dans la littérature, telles que des techniques d'ultra-filtration, de filtration et de centrifugation, ou de précipitation au polyéthylène glycol, ces techniques pouvant être combinées entre elles ou à des techniques de gel-filtration, de chromatographie sur sulfate de cellulose ou sur sulfate de polysaccharide réticulé (JP-B-65 000 611, JP-A-53 133 627, JP-A-50 048 118, JP-A-2 223 531, US-A-4 725 546, JP-A-49 020 322 et B-81 005 204, JP-B-67 025 408).

Dans l'art antérieur, les préparations virales sont inactivées, ainsi que le recommande l'Organisation Mondiale de la Santé, par des agents chimiques tels que le formol selon des procédures standardisées, à savoir inactivation de longue durée pendant cinquante à soixante jours à +4°C à une concentration de formol de 1/2000, en raison de l'instabilité des virus à des températures plus élevées dans cet environnement.

Les vaccins du commerce ainsi obtenus sont efficaces mais sont difficiles et coûteux à préparer, purifier et inactiver. Ils peuvent, en outre, conduire à des réactions secondaires dues aux contaminants provenant des tissus de souriceaux, ce qui en limite, parfois, l'application.

Il est donc souhaitable de pouvoir produire un vaccin par d'autres techniques de nature plus industrielle et notamment en utilisant une multiplication et une propagation du virus sur lignée cellulaire. Toutefois, la production d'un vaccin en grandes quantités, par des méthodes très industrialisées, est beaucoup plus difficile que dans le cas de la multiplication par voie intracrânienne, et ceci non seulement en raison des quantités importantes qui doivent être traitées, mais également des difficultés d'obtenir et de contrôler des rendements élevés, ainsi que des problèmes de purification posés par les contaminants pouvant provenir des cellules ou du milieu de multiplication virale que sont alors rencontrés.

Il est connu dans l'art antérieur que le virus de l'encéphalite japonaise peut se propager sur diverses cultures de cellules, y compris sur des cultures de lignées cellulaires, notamment des cellules Vero. Cependant, les méthodes de culture divulguées ne permettent pas d'obtenir des rendements satisfaisants dans des conditions de culture industrielle à grande échelle, qui seule permet une production à un coût raisonnable. Il n'est pas non plus décrit, dans l'art antérieur, de méthode pour purifier à un haut degré les préparations virales provenant de la propagation et de la multiplication sur lignées cellulaires.

L'invention se propose donc de remédier à ces inconvénients et de fournir un procédé de production d'un vaccin contre l'encéphalite japonaise, qui puisse être mis en oeuvre à grande échelle dans des conditions sûres, rapides et économiques et qui permette d'obtenir un vaccin, efficace d'une très grande pureté, avec un très bon rendement industriel.

Pour atteindre ces buts, l'invention a pour objet un procédé de production industrielle d'un vaccin contre l'encéphalite japonaise, caractérisé en ce qu'il comprend les étapes suivantes:
a) mise en culture de cellules provenant d'une lignée cellulaire,
b) inoculation de la culture de cellules obtenues par du virus de l'encéphalite japonaise en présence d'un milieu de multiplication virale,
c) propagation et multiplication du virus sur les cellules,
d) récolte du milieu de multiplication virale constituant une suspension de virus produits par les cellules,
e) purification de la suspension virale par au moins une étape de chromatographie échangeuse d'ions, une étape de chromatographie d'adsorption et une étape de perméation de gel,
f) formulation et mise sous forme pharmaceutique de la suspension virale pour assurer sa conservation jusqu'à son utilisation.

Selon une caractéristique particulière de l'invention, la quantité de virus inoculée correspond à une multiplicité d'infection inférieure à 0,1. Ainsi, on obtient un bon rendement pour la propagation et la multiplication virale.

Selon une caractéristique particulière de l'invention, le procédé comprend, en outre, une étape d'inactivation de la suspension virale avant ou après l'étape de purification e). Il est ainsi possible de fabriquer un vaccin inactivé tout en utilisant pour la propagation et la multiplication virale une souche virulente.

Selon une caractéristique du procédé de l'invention, l'inactivation est réalisée au moyen d'un agent chimique à température ambiante. Ainsi, l'inactivation se produit rapidement.

Selon un mode particulier de réalisation, le procédé consiste à utiliser une lignée de cellules Vero pour assurer la multiplication vitale. Il est ainsi possible d'obtenir de bons rendements car ces cellules sont très permissives au virus de l'encéphalite japonaise.

Selon une autre caractéristique de réalisation de l'invention, le procédé consiste à purifier la suspension virale en effectuant les étapes suivantes :
. chromatographie par échange d'ions,
. chromatographie d'adsorption,
. perméation de gel.

Il est, ainsi, possible d'obtenir un vaccin à très haut degré de pureté.

Selon un autre mode de réalisation de l'invention, le procédé consiste, en outre, après l'étape de récolte d), à introduire à nouveau du milieu de multiplication virale neuf, à attendre un temps suffisant pour permettre à nouveau la multiplication du virus et à effectuer une nouvelle récolte du milieu de multiplication virale.

On peut, ainsi, obtenir un nombre important de récoltes à partir d'une même culture cellulaire.

Il est également décrit un vaccin obtenu par culture sur cellules provenant d'une lignée cellulaire caractérisé en ce qu'il comporte du virus de l'encéphalite japonaise et en ce que la teneur en ADN cellulaire est inférieure à 100 pg/dose.

Un tel vaccin présente à la fois l'efficacité et la sûreté nécessaire tant du point de vue des contaminants viraux que des protéines pour être administré de façon systématique à toute personne susceptible d'être en contact avec le virus.

D'autres objets et avantages de la présente invention apparaîtront à la lecture de la description qui va suivre.

Selon l'invention, on peut effectuer la culture cellulaire, soit en fermenteur, soit de façon traditionnelle en flacons (boîtes de Roux, flacons roulants, Multiray^{™}, Cell-Cube^{™}...).

De préférence, cependant, on dispose d'un fermenteur de grand volume (500 à 2000 l) contenant des microporteurs ainsi qu'un milieu de culture cellulaire, dans lequel on introduit un inoculum de cellules d'une lignée cellulaire permissive au virus de l'encéphalite japonaise ; il peut s'agir de cellules BHK 21 (Baby Hamster Kidney) ou encore de cellules Vero.

Les microsupports permettant la culture en suspension dans le fermenteur peuvent être différents microsupports déjà connus pour un tel usage ; on peut notamment citer les particules Cytodex 1^{™} à une concentration de 1 à 3 g/l de milieu de culture comme convenant particulièrement bien à la culture de cellules Vero. Les durées, températures et autres conditions de culture, et notamment la composition du milieu de culture sont adaptées en fonction de la nature des cellules Vero sur microsupports de Cytodex 1^{™}, une durée de 4 jours était appropriée pour obtenir une bonne croissance cellulaire et permettre ainsi d'inoculer le virus avant la phase stationnaire de la culture. On remplace ensuite le milieu de culture par un milieu de multiplication virale et on introduit dans le fermenteur un inoculum de virus de l'encéphalite japonaise avec une quantité calculée pour avoir une faible valeur de la multiplicité d'infection (ou MOI pour Multiplicity of Infection) qui est le rapport de la quantité de particules virales introduites sur le nombre de cellules présentes.

Cette multiplicité est de préférence inférieure à environ 0,1 et de préférence encore inférieure à 0,01.

La souche virale utilisée peut être une souche atténuée telle que la souche SA 14-14-2 ou toute souche immunogène virulente connue telle que la souche Nakayama ou Beijing. La souche P₃ au 88ème passage fournie par le NVSI (National Vaccine and Serum Institute, Beijing) convient bien aux besoins de l'invention.

Le milieu utilisé pour la multiplication virale est un milieu habituel, tel que le MEM, dans lequel il est important que la quantité de protéines soit réduite le plus possible. On utilise de préférence un milieu dont la concentration en protéines, qui est habituellement de l'albumine humaine, est inférieure à 5 g/l. De préférence même, on utilise un milieu de culture complètement dépourvu en protéines.

La durée nécessaire à la propagation et à la multiplication virale peut être déterminée par la surveillance du titre infectieux. On considère qu'une récolte de virus peut être effectuée lorsque le titre LD 50/ml est de l'ordre de 10⁷ ou 10⁸. La récolte est effectuée par simple prélèvement du milieu de multiplication virale qui contient les virus produits par les cellules. De façon avantageuse, après avoir prélevé le milieu de multiplication virale, on introduit à nouveau dans le fermenteur du milieu neuf afin de permettre une nouvelle multiplication du virus conduisant à une nouvelle récolte. On peut, ainsi, obtenir facilement jusqu'à 8 récoltes successives dans le même fermenteur à partir de la même culture cellulaire. La durée nécessaire à la propagation et à la multiplication du virus pour obtenir une dose LD 50/ml de 10⁷ ou 10⁸ est généralement de 2 à 3 jours ; on réalise de préférence une première récolte 3 jours après l'inoculation virale, puis des récoltes successives tous les 2 ou 3 jours.

Ainsi, un cycle complet dans un fermenteur, peut durer 23 jours répartis de la façon suivante :
. J0 : démarrage de la culture de cellules sur les microsupports dans le fermenteur,
. J4 : remplacement du milieu de culture cellulaire par du milieu de multiplication virale et inoculation du virus,
. J7 : première récolte,
. J9 : deuxième récolte,
. J11 : troisième récolte,
. J14 : quatrième récolte,
. J16 : cinquième récolte,
. J18 : sixième récolte,
. J21 : septième récolte,
. J23 : huitième récolte.

Les différentes récoltes obtenues peuvent ensuite être traitée séparément ou en mélange.

Le traitement consiste soit en une purification et une inactivation, l'inactivation pouvant être réalisée avant ou après l'étape de purification.

L'inactivation est une étape indispensable au procédé selon l'invention lorsque la souche virale utilisée au départ est une souche virulente ; par contre, lorsque la souche utilisée pour la multiplication virale est une souche atténuée telle que la souche SA 14-14-2, cette étape d'inactivation peut être soit omise, soit réalisée afin d'être dans les meilleures conditions de sécurité possibles.

Selon l'invention, on effectue l'inactivation au moyen d'agents chimiques à une température ambiante. Par température ambiante selon l'invention, on entend une température largement supérieure à + 4°C qui est la température habituellement utilisée par l'inactivation du virus JEV. Cette température peut avantageusement être comprise entre 20 et 37°C et on préfère une température de l'ordre de 25°C. On a, en effet, constaté que le virus est rapidement inactivé à cette température et on a, en outre, pu noter que, de façon surprenante, le virus contenu dans son milieu de multiplication virale, est stable malgré la température élevée ; cette durée très courte de l'inactivation est un avantage important du procédé de l'invention d'un point de vue industriel.

Les agents chimiques utilisés pour l'inactivation peuvent notamment être le formol ou la bétapropiolactone ; on préfère le formol. On peut, par exemple, effectuer l'inactivation à l'aide de formol à 25 ou à 37°C pendant 14 jours ou moins ; de préférence, la durée sera d'au moins 7 jours. De façon avantageuse selon l'invention, la concentration de formol utilisé peut être inférieure à celle classiquement utilisée dans l'inactivation du virus JEV ; elle peut, par exemple, être de l'ordre de 1/2000 à 1/8000 et notamment de 1/4000.

Il est possible également de procéder successivement à 2 étapes d'inactivation différentes mettant par exemple en oeuvre 2 agents chimiques différents.

On peut également, avant cette étape d'inactivation, procéder à la filtration de chaque récolte afin d'éliminer les débris cellulaires (protéines, acides nucléiques...), ainsi qu'à sa concentration de façon à augmenter le titre en virus et la teneur en protéines du milieu liquide. Le facteur de concentration est de préférence au moins égal à 10, par exemple de l'ordre de 1000. La concentration peut être effectuée par les moyens habituels et notamment l'ultrafiltration.

La suspension virale, inactivée ou non selon le procédé utilisé, doit être purifiée. Selon une caractéristique importante de l'invention, l'étape de purification comprend au moins une chromatographie. De façon avantageuse, on procède successivement aux 3 étapes suivantes :
. chromatographie échangeuse d'ions,
. chromatographie d'adsorption par chélation,
. perméation de gel.

La chromatographie échangeuse d'ions est de préférence une chromatographie échangeuse d'anions, qu'ils soient faibles ou forts. Le support utilisé est par exemple le DEAE-Spherodex™ (vendu par Biosepra, USA) qui retient sélectivement les particules virales et laisse passer l'essentiel des protéines contaminantes.

L'éluat contenant les virus peut ensuite être effectuée sur des supports tels que l'hydroxyapatite ou les gels chelatants (calcium...). Dans ce cas, le virus ne se fixe pas sur le support qui retient plus spécialement les acides nucléiques.

A la suite de cette étape, on procède à une gel filtration ou tamisage moléculaire (encore appelé perméation de gel) sur tout support approprié tel que le Sepharose 6FF™ (Pharmacia) ou le Fractogel™ (E. Merck). Lors de cette opération, l'élution permet de récupérer les particules virales dans la première fraction ; les pics d'élution suivants correspondent aux protéines virales et aux impuretés résiduelles. On conserve donc uniquement, pour la fabrication d'un vaccin, la première fraction de l'éluat.

De façon avantageuse, on peut procéder, entre la chromatographie d'adsorption et la gel filtration, à une étape de concentration, de préférence par ultrafiltration avec une membrane dont le seuil de coupure est de 10 000 daltons.

La suspension virale purifiée et éventuellement inactivée est ensuite formulée pour obtenir le titre antigénique désiré ; on peut également lui ajouter un stabilisant ou un adjuvant ; elle est ensuite mise sous forme pharmaceutique afin d'être conservée dans de bonnes conditions jusqu'à son utilisation.

### Exemple.

### 1. Matériels

- cellules Vero : les cellules Vero utilisées pour inoculer le fermenteur proviennent d'une banque cellulaire Vero au 137ème passage, banque ayant subi tous les contrôles nécessaires à sa caractérisation et à sa qualification.
- Le virus JEV : la souche virale utilisée est la souche P₃ au 88ème passage fournie par le NVSI.

Une ampoule de virus reçue est mise en suspension dans 100 ml de milieu et filtrée à l'aide d'un filtre 0,1 µm. La solution est utilisée pour infecter deux flacons de 75 cm². Cinq jours après, le surnageant recueilli est filtré (filtre de 0,2 µm). Plusieurs récoltes ont été faites et le mélange formant le lot de semence primaire présente un titre LD 50/ml = 10^{8,16}. On prépare le lot de semence de travail à raison de 10 ml de lot de semence primaire pour infecter 12 flacons roulants de 850 cm². Plusieurs récoltes peuvent être effectuées et le mélange de 30,2 litres a un titre LD 50/ml = 10^{8,31}.

Les lots de semence primaire et de travail sont ensuite contrôlés afin d'assurer leur caractérisation et leur qualification.

### 2. Procédé du culture

On remplit la cuve d'un fermenteur de 500 l, avec un milieu de culture usuel pour cellules Vero contenant des microsupports Cytodex 1™ à une concentration de 3 g/l. On ensemence le milieu par un inoculum de cellules Vero (200 000 cellules/ml).

On laisse les cellules se fixer et croître pendant quatre jours. A la fin des quatre jours, le milieu de culture est remplacé par un milieu de multiplication virale.

Un inoculum de virus est introduit dans la cuve avec une quantité de virus calculée pour avoir une multiplicité d'infection MOI égale à 1/500. Les différentes cultures sont effectuées à la température de 37°C. Trois jours après l'inoculation virale, on recueille le milieu de multiplication virale qui fournit la première récolte. Le milieu de multiplication virale est remplacé par un milieu neuf et une nouvelle récolte est effectuée tous les deux jours, le nombre total de récoltes étant égal à six.

Le tableau I montre les titres obtenus à chaque récolte.

| **Récoltes n°** | **R1** | **R2** | **R3** | **R4** | **R5** | **R6** |
|---|---|---|---|---|---|---|
| Jours après infection | 3 | 5 | 7 | 10 | 12 | 14 |
| Titre LD 50/ml | 10^{8,4} | 10^{8,4} | 10^{8,5} | 10^{8,1} | 10^{8,2} | 10^{7,3} |

Les récoltes sont filtrées sur filtre membrane (diamètre des pores : 0,2 µm).

L'ensemble des récoltes est mélangé.

Le mélange de récolte est concentré d'un facteur 100 par ultrafiltration sur membrane 10 000 daltons.

On ajoute au mélange de récolte concentré une solution de formol à une concentration finale de 1/4000 et on maintient à température ambiante (de 20 à 25°C) sous agitation continue pendant 14 jours.

On procède ensuite à une nouvelle filtration et on vérifie l'inactivation par un contrôle en 2 étapes selon le protocole de l'OMS (Rapport technique 771 de 1988).

Toutes les préparations inactivées par le formol se sont montrées satisfaisantes.

La solution inactivée est passée sur une colonne chromatographique d'échange d'ions à groupement DEAE équilibrée à pH 8, (résine DEAE-Sphérodex^{™}). Le virus est retenu sur la colonne. Après lavage par un tampon phosphate, le virus est élué par un tampon phosphate, NaCl 0,2M. La majorité des contaminants protéiques sont ainsi éliminés.

L'éluat est purifié par affinité de chélation par interaction calcique en injectant l'éluat à travers une colonne de Chelating Sepharose^{™} (Pharmacia). Le virus n'est pas fixé et traverse directement la colonne.

On procède ensuite à une concentration par ultrafiltration sur des membranes dont le seuil de coupure est de 10 000 Da pour réduire le volume de la suspension virale d'un facteur d'environ 20.

La solution concentrée de virus prépurifiée est introduite dans une colonne de Sepharose 6 FF^{™}. On procède à une élution par un tampon phosphate NaCl 0,2M. Les particules virales se trouvent éluées dans la fraction exclue.

Les caractéristiques du procédé de purification figurent sur le tableau II.

**Tableau II**

| **Etape** | **DEAE** | **Chélation** | **Gel filtration** |
|---|---|---|---|
| Protéines résiduelles (*) | 50 % | 100 % | 0,4 % |
| Teneur en ADN (pg/ml) | < 7000 | < 30 | < 30 |
| Virus récupéré (*) (ELISA protéine E) | 90 % | 76 % | 20 % |

| | | | |
|---|---|---|---|
| (*) Rendements étapes par étape | | | |

La solution virale inactivée purifiée a été utilisée pour immuniser des souris par injection au jour 0 puis au jour 7. L'épreuve par le virus virulent est effectuée à l'aide d'une solution à 10⁵ LD 50/ml au jour 30. Toutes les souris vaccinées par la préparation purifiée non diluée et par la préparation purifiée diluée au 1/32 ont été protégées. Dans le même test, les souris vaccinées par le vaccin Biken (dilution 1/32) ont été éprouvées et seules les 2/5 ont été protégées.

## Revendications

1. Procédé de production industrielle d'un vaccin contre l'encéphalite japonaise, **caractérisé en ce qu'**il comprend les étapes suivantes :
a) mise en culture de cellules provenant d'une lignée cellulaire,
b) inoculation de la culture de cellules obtenue par du virus de l'encéphalite japonaise en présence d'un milieu de multiplication virale,
c) propagation et multiplication du virus sur les cellules,
d) récolte du milieu de multiplication virale constituant une suspension de virus produits par les cellules,
e) purification de la suspension virale par au moins une étape de chromatographie échangeuse d'ions, une étape de chromatographie d'adsorption et une étape de perméation de gel,
f) formulation et mise sous forme pharmaceutique de la suspension virale pour assurer sa conservation jusqu'à son utilisation.

2. Procédé selon la revendication 1, **caractérisé en ce que** la quantité de virus inoculée correspond à une multiplicité d'infection (MOI) inférieure à 0,1.

3. Procédé selon la revendication 1 ou 2, **caractérisé en ce qu'**il comprend, en outre, une étape d'inactivation de la suspension virale avant ou après l'étape de purification e).

4. Procédé selon la revendication précédente, **caractérisé en ce que** l'inactivation est réalisée au moyen d'agent chimique à température ambiante.

5. Procédé selon une des revendications précédentes, **caractérisé en ce que** les cellules utilisées pour la multiplication virale sont des cellules VERO.

6. Procédé selon une des revendications précédentes, **caractérisé en ce que** la purification est effectuée grâce aux opérations suivantes :
. chromatographie par échange d'ions,
. chromatographie d'adsorption par chélation,
. perméation de gel.

7. Procédé selon une des revendications précédentes, **caractérisé en ce qu'**il consiste en outre, après l'étape de récolte d), à introduire à nouveau du milieu de multiplication virale neuf, à attendre un temps suffisant pour permettre à nouveau la multiplication du virus, et à effectuer une nouvelle récolte du milieu de multiplication virale.

8. Procédé selon la revendication 7, **caractérisé en ce qu'**il consiste à récolter le milieu de multiplication virale et à le remplacer par du milieu neuf, un nombre de fois compris entre 1 et 7.

9. Procédé selon une des revendications précédentes, **caractérisé en ce qu'**il consiste en outre, après l'étape d) à filtrer la suspension virale récoltée.

10. Procédé selon une des revendications précédentes, **caractérisé en ce que** le milieu de multiplication virale possède une concentration en protéines inférieure à 5 g/l.

## Claims

1. Process for the industrial production of a vaccine against Japanese encephalitis, **characterised in that** it comprises the following steps:
a) culturing of cells originating from a cell line,
b) inoculation of the cell culture obtained by Japanese encephalitis virus in the presence of a viral multiplication medium,
c) propagation and multiplication of the virus on the cells,
d) harvesting of the viral multiplication medium constituting a suspension of viruses produced by the cells,
e) purification of the viral suspension by at least one ion-exchange chromatographic step, an adsorption chromatographic step and a gel permeation step,
f) formulation and incorporation of the viral suspension in a pharmaceutical form in order to ensure its conservation up to the time of its use.

2. Process according to Claim 1, **characterized in that** the amount of virus inoculated corresponds to a multiplicity of infection (MOI) of less than 0.1.

3. Process according to Claim 1 or 2, **characterized in that** it also comprises a step of inactivation of the viral suspension before or after the purification step e).

4. Process according to the preceding claim, **characterized in that** the inactivation is performed using a chemical agent at room temperature.

5. Process according to one of the preceding claims, **characterized in that** the cells used for the viral multiplication are Vero cells.

6. Process according to one of the preceding claims, **characterized in that** the purification is carried out by means of the following operations:
. ion-exchange chromatography,
. adsorption chromatography by chelation,
. gel permeation.

7. Process according to one of the preceding claims, **characterised in that** it also consists, after the harvesting step d), in reintroducing new viral multiplication medium, in waiting for a sufficient period to allow further multiplication of the virus and in carrying out further harvesting of the viral multiplication medium.

8. Process according to Claim 7, **characterized in that** it consists in harvesting the viral multiplication medium and in replacing it by new medium, a number of times between 1 and 7.

9. Process according to one of the preceding claims, **characterized in that** it also consists, after step d), in filtering the viral suspension harvested.

10. Process according to one of the preceding claims, **characterized in that** the viral multiplication medium possesses a protein concentration of less than 5 g/l.

## Patentansprüche

1. Verfahren zur industriellen Herstellung einer Vakzine gegen Japanische Encephalitis, **dadurch gekennzeichnet, dass** es folgende Stufen umfasst:
a) Kultur von Zellen, die von einer Zelllinie stammen,
b) Inokulieren der erhaltenen Zellkultur mit dem Virus der Japanischen Encephalitis in Anwesenheit eines viralen Multiplikationsmediums,
c) Propagation und Multiplikation des Virus an den Zellen,
d) Ernte des viralen Multiplikationsmediums, das eine Suspension des Virus, erzeugt durch die Zellen, bildet,
e) Reinigung der viralen Suspension mit mindestens einer Ionenaustauscherstufe, einer Adsorptionschromatographie-Stufe und einer Gelpermeations-Stufe,
f) Formulierung und Bringen der viralen Suspension in eine pharmazeutische Form, um eine Konservierung bis zur Verwendung sicherzustellen.

2. Verfahren nach Anspruch 1, **dadurch gekennzeichnet, dass** die Menge des inokulierten Virus einem Vielfachen der Infektion (MOI) unter 0,1 entspricht.

3. Verfahren nach Anspruch 1 oder 2, **dadurch gekennzeichnet, dass** es darüber hinaus eine Inaktivierungsstufe der viralen Suspension vor oder nach der Reinigungsstufe e) umfasst.

4. Verfahren gemäß dem vorhergegangenen Anspruch , **dadurch gekennzeichnet, dass** die Inaktivierung mit einem chemischen Mittel bei Raumtemperatur durchgeführt wird.

5. Verfahren nach einem der vorhergehenden Patentansprüche, **dadurch gekennzeichnet, dass** die zur viralen Multiplikation verwendeten Zellen VERO-Zellen sind.

6. Verfahren nach einem der vorhergehenden Ansprüche, **dadurch gekennzeichnet, dass** die Reinigung durch folgende Arbeitsgänge erzielt wird:
• lonenaustauscherchromatographie,
• Adsorptionschromatographie durch Chelieren,
• Gelpermeation.

7. Verfahren nach einem der vorhergehenden Ansprüche, **dadurch gekennzeichnet, dass** man außerdem nach der Erntestufe d) erneut in ein neues virales Multiplikationsmedium einführt, eine ausreichende Zeit verstreichen lässt, um die Multiplikation des Virus erneut zu ermöglichen und eine neue Ernte des viralen Multiplikationsmediums durchführt.

8. Verfahren nach Anspruch 7, **dadurch gekennzeichnet, dass** es darin besteht, das virale Multiplikationsmedium zu ernten und zwischen ein- und siebenmal durch ein neues Medium zu ersetzen.

9. Verfahren nach einem der vorhergehenden Ansprüche, **dadurch gekennzeichnet, dass** es außerdem darin besteht, nach der Stufe d) die geerntete virale Suspension zu filtrieren.

10. Verfahren nach einem der vorhergehenden Ansprüche, **dadurch gekennzeichnet, dass** das virale Multiplikationsmedium eine Proteinkonzentration unter 5 g/l aufweist.
